# EUROPEAN PATENT SPECIFICATION

(11) **EP 3 106 078 B1**
(45) Date of publication and mention of the grant of the patent: **15.05.2019**
(21) Application number: 15749657.1
(22) Date of filing: 12.02.2015
(51) Int. Cl.: A61B 1/00, A61B 1/01, A61M 16/04, A61B 17/34

(54) **OVER-TUBE DEVICE FOR ENDOSCOPES**
ÜBERROHRVORRICHTUNG FÜR ENDOSKOPE
DISPOSITIF SUR-TUBE POUR ENDOSCOPES

(30) Priority: 12.02.2014 JP 2014024937
(43) Date of publication of application: 21.12.2016
(73) Proprietor: KOHAN CO., LTD., Kobe-shi, Hyogo 651-0061 (JP); Osaka University, Suita-shi, Osaka 565-0871 (JP)
(72) Inventor: NAKAJIMA, Kiyokazu, Suita-shi Osaka 565-0871 (JP); HIROTA, Masashi, Suita-shi Osaka 565-0871 (JP); KATO, Motohiko, Suita-shi Osaka 565-0871 (JP); UMEHARA, Satoru, Kobe-shi Hyogo 651-0061 (JP); KURIYAMA, Shinichi, Kobe-shi Hyogo 651-0061 (JP); KAWAI, Naoki, Osaka-shi Osaka 543-0035 (JP); DEGUCHI, Osamu, Tokyo 120-0035 (JP); ITO, Yuya, Tokyo 120-0035 (JP)
(74) Representative: Vigand, Philippe
(86) International application number: PCT/JP2015/053869
(87) International publication number: WO 2015/122474

(56) References cited:
- EP-A1- 1 800 596
- WO-A1-2009/119053
- WO-A1-2013/146727
- JP-A- H07 163 516
- JP-A- H10 234 656
- JP-A- 2000 245 687
- JP-A- 2000 245 687
- JP-A- 2000 245 688
- JP-A- 2000 245 688
- US-A1- 2001 049 509

## Description

### Technical Field

The present invention relates to an over-tube device for endoscope, and more particularly, to an over-tube device for endoscope that is capable of preventing withdrawal of the over-tube during medical treatment of the patient.

### Background Art

Conventionally, in an endoscopic therapy in a digestive tract lumen such as a stomach and an esophagus, for example, for the purpose of safe guidance of endoscope and for the auxiliary purpose of the endoscopic therapy, "over-tubes" which surround the endoscope to be inserted into the digestive tract lumen have been used. Further, among the over-tubes, in order to prevent a withdrawal during use, there is an over-tube fixed to a tool called a byte block used to protect oral mucosa and teeth of a patient.

Furthermore, for the purpose of development of a new endoscopic therapy, an over-tube of the type that has a conduit "side channel" for inserting a forceps or the like into a tubular wall, and is capable of rotating the tube around the endoscope in a state of inserting the forceps into the side channel is also suggested (Patent Literature 1).

By such an over-tube, the grasping forceps can be operated completely independent from the endoscope, and a new endoscopic therapy (procedure) that has not been achieved before can be realized.

However, in order to more securely and safely perform the procedure, it has been desired to extract or insert the over-tube along the axial direction without interfering the rotation property, for example, in a range from several cm to about 10 cm. Meanwhile, there is a need to avoid the risk that the over-tube is excessively inserted into the lumen of the patient or falls out of the patient by such extraction and insertion.

It was difficult to achieve a procedure that enables adequate insertion and movement into the lumen of the patient, using a fixing mechanism made up of a combination of the conventional bite block and the over-tube.

### Citation List

### Patent Literature

Patent Literature 1: WO 2011/004820 A
Patent Literature 2: JP 2000245687 A
Patent Literature 3: JP 2000245688 A

### Summary of Invention

### Technical Problem

The invention, which is defined in independent claim 1 and the dependent claims 2-7, has been made to solve the aforementioned problems, and an object thereof is to provide an over-tube device for endoscope that allows free extraction and insertion of the over-tube within a limited range, while maintaining the rotation ability of the over-tube, and prevents extraction and insertion (withdrawal) of the over-tube of an unintended range of an operator.

### Solution to Problem

The present invention is an over-tube device for endoscope including: a tube body that has a smooth outer surface capable of rotating within a lumen of a patient, and includes a channel for inserting the endoscope thereinto; a stopper ring mounted to surround a side portion of the tube body; and a rotation lock ring that is mounted to surround the side portion of the tube body and to cover the stopper ring, and is capable of sliding in an axial direction of the tube body, the rotation lock ring including: a connecting section for being connected to a bite block; and a hook that is capable of being locked on a proximal end side of the stopper ring extending from a base portion.

In an embodiment, the tube body is provided with a proximal end side stopper at a proximal end, and the hook of the rotation lock ring is slidable from the stopper ring to the proximal end side stopper.

In an embodiment, the outer surface of the tube body has a substantially cylindrical shape.

In an embodiment, the hook extending from the base portion of the rotation lock ring constitutes a leaf spring.

In an embodiment, the tube body includes a second channel for inserting the treatment tool.

Furthermore, in the present invention, the tube body may include a proximal end side stopper at a proximal end thereof, and a second channel for inserting a treatment tool, the proximal end side stopper may have a treatment tool insertion port as an opening capable of inserting the treatment tool into the second channel by inserting the treatment tool, and an endoscope insertion port as an opening capable of inserting the endoscope into the channel by inserting the endoscope, and the treatment tool insertion port and the endoscope insertion port may be provided to be inclined to each other so that a gap between axes thereof becomes large toward the proximal side.

In addition, in the present invention, the treatment tool insertion port may be provided with a degassing prevention valve that prevents degassing through the second channel.

In addition, in the present invention, the tube body may further include a proximal end side stopper at a proximal end thereof, the proximal end side stopper may have an endoscope insertion port as an opening capable of inserting the endoscope into the channel by inserting the endoscope, and the endoscope insertion port may be configured to mount a degassing prevention adapter for preventing degassing through the channel, and may have a lock mechanism that prevents the mounted degassing prevention adapter from being detached from the endoscope insertion port.

### Advantageous Effects of Invention

According to the invention, it is possible to allow the free extraction and insertion of the over-tube within the limited range, while maintaining the rotation ability of the over-tube, and to prevent withdrawal of the over-tube of the unintended range of the operator. Thus, the operator is able to perform more advanced and innovative endoscopic therapy using the over-tube, without any fear of withdrawal of the over-tube.

### Brief Description of Drawings

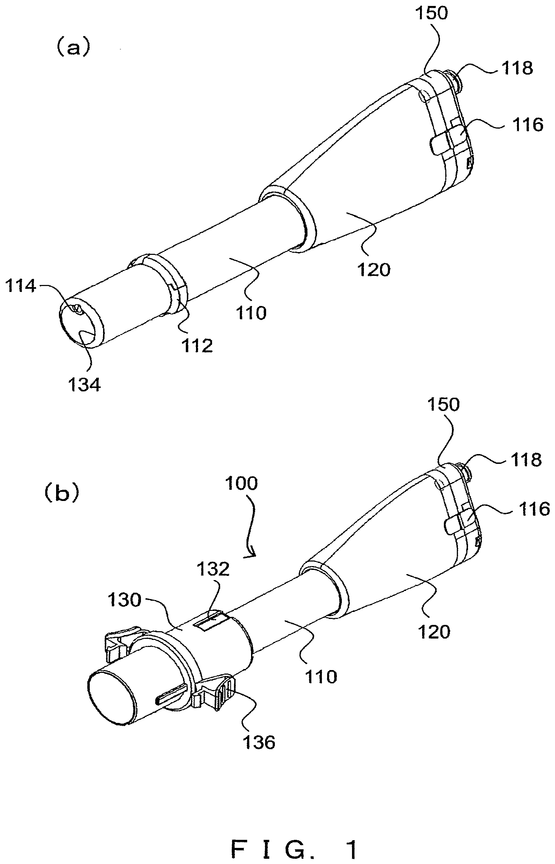

Figs. 1(a) and 1(b) are diagrams for schematically illustrating an over-tube device for endoscope of the invention, in which Fig. 1(a) is a perspective view of a state in which a stopper ring and a proximal end side stopper (end cover) are mounted to a tube body, and Fig. 1(b) is a perspective view of a state in which a rotation lock ring is further mounted to the tube body.

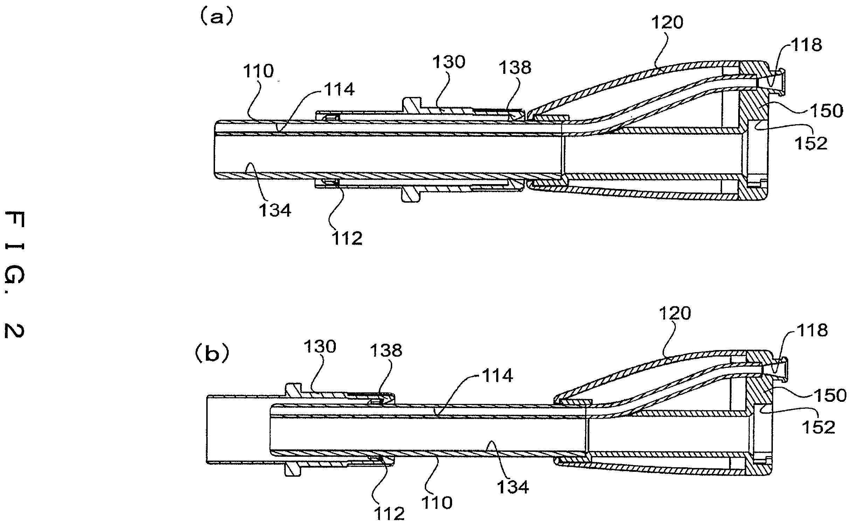

Figs. 2(a) and 2(b) are diagrams for schematically illustrating a sliding state of the tube body of the over-tube device for endoscope, in which Fig. 2(a) is a cross-sectional view illustrating a state in which a distal end side of a proximal end side stopper (end cover) of the tube body is in contact with the proximal end of the rotation lock ring, and Fig. 2(b) is a cross-sectional view of a state in which the tube body is locked at the proximal end side of the stopper ring mounted to the tube body by the rotation lock ring hook.

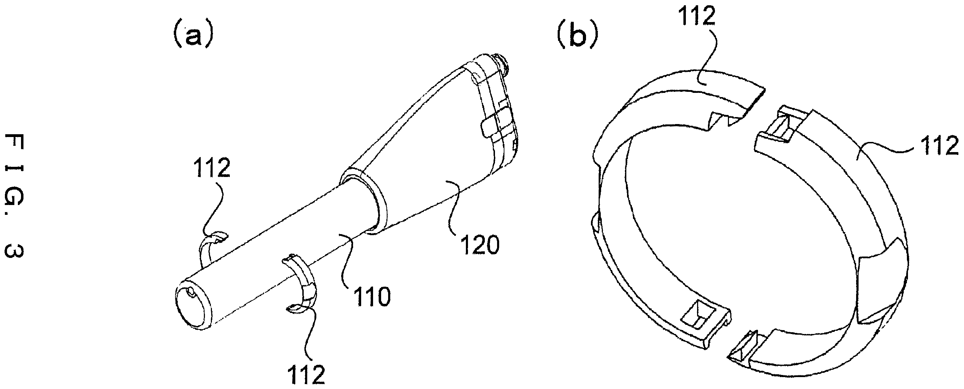

Figs. 3(a) and 3(b) are diagrams for schematically illustrating mounting of the stopper ring to the tube body, in which Fig. 3(a) is a perspective view of the tube body and the stopper ring, and Fig. 3(b) is an enlarged view illustrating the structure of the stopper ring.

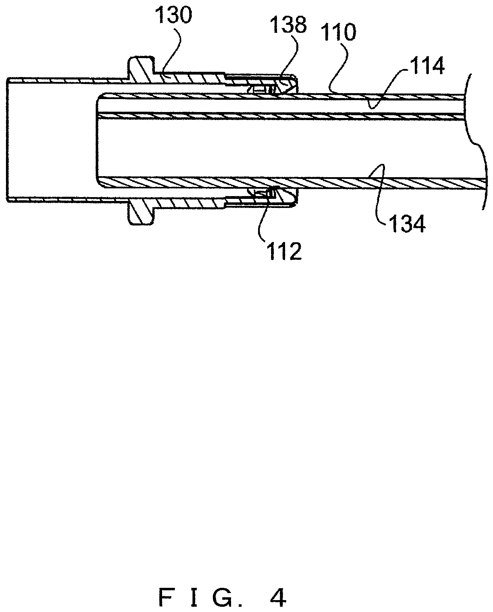

Fig. 4 is a diagram for schematically illustrating a state of mounting the rotation lock ring to the tube body, and a cross-sectional view of the rotation lock ring and the tube body.

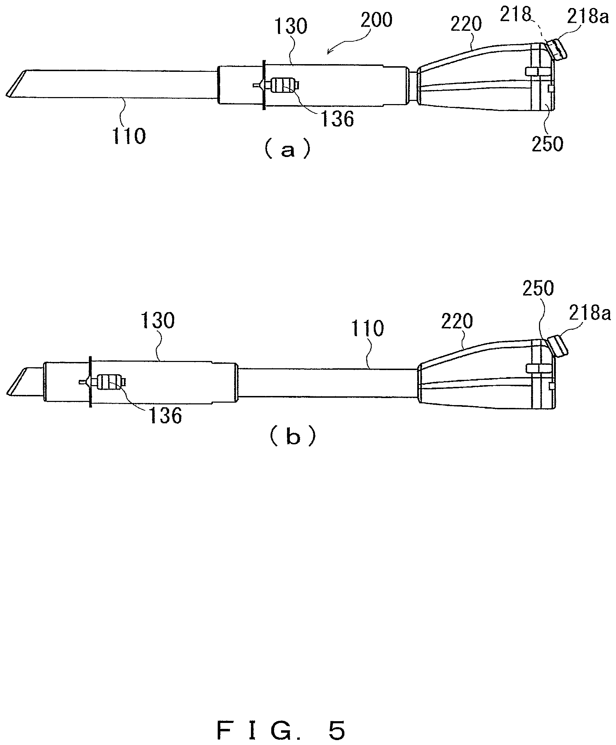

Figs. 5(a) and 5(b) are diagrams for schematically illustrating an over-tube device for endoscope according to a second embodiment of the invention, in which Fig. 5(a) is a side view of a case where the rotation lock ring relatively moves to the proximal end side of the tube body, and Fig. 5(b) is a side views of a case where the rotation lock ring relatively moves to the distal end side of the tube body.

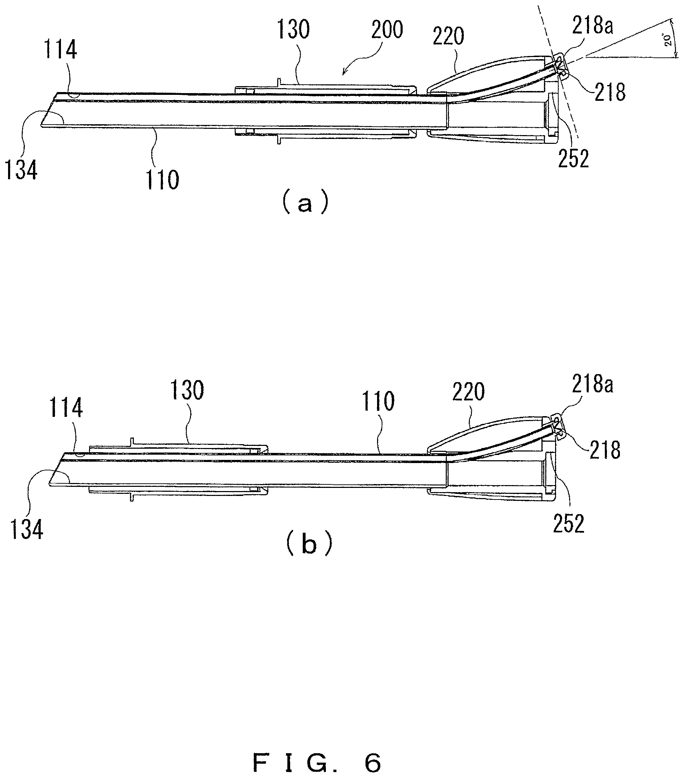

Figs. 6(a) and 6(b) are diagrams for schematically illustrating the over-tube device for endoscope according to the second embodiment of the present invention, in which Fig. 6(a) is a cross-sectional view of a case where the rotation lock ring relatively moves to the proximal end side of the tube body, and Fig. 6(b) is a cross-sectional view of a case where the rotation lock ring relatively moves to the distal end side of the tube body.

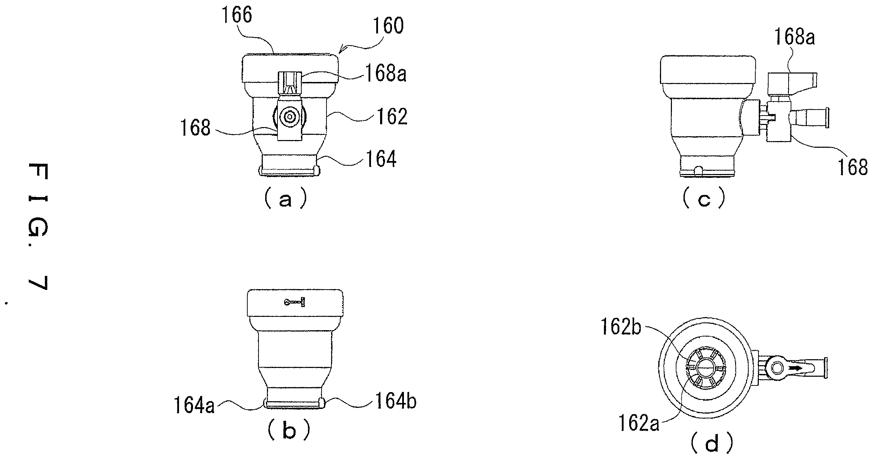

Figs. 7(a) to 7(d) are diagrams illustrating an exterior of a degassing prevention adapter according to the second embodiment of the invention, in which Figs . 7(a) to 7(c) are side views of the degassing prevention adapter, and Fig. 7(d) is a plan view of the degassing prevention adapter.

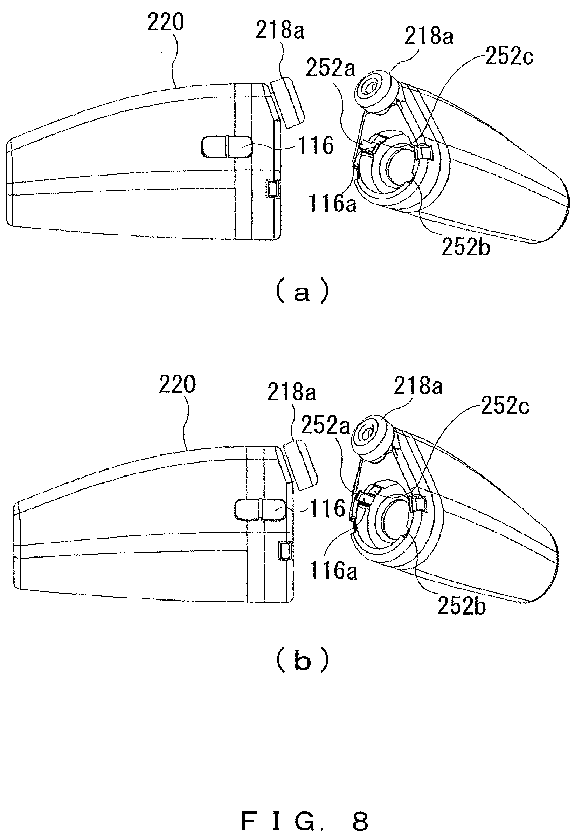

Figs. 8(a) and 8(b) are diagrams illustrating an operation of the stopper provided at the proximal end of a handling connector according to the second embodiment of the invention, in which Fig. 8(a) is a diagram illustrating a state of the vicinity of an opening portion of the handling connector when the stopper is turned OFF, and Fig. 8(b) is a diagram illustrating a state of the vicinity of the opening portion of the handling connector when the stopper is turned ON.

### Description of Embodiments

Hereinafter, the invention will be described with reference to the drawings.

### <First Example>

Figs. 1(a) and 1(b) are diagrams for schematically illustrating an over-tube device 100 for endoscope of the invention, Fig. 1(a) is a perspective view of a state in which a stopper ring 112 and a proximal end side stopper (end cover) 120 are mounted to a tube body 110, and Fig. 1(b) is a perspective view of a state in which a rotation lock ring 130 is further mounted to the tube body 110.

Here, unless otherwise specified, the term "endoscope" as used in this specification refers to a medical flexible endoscope. Such a flexible endoscope uses a flexible material, and for example, there are an endoscope using glass fibers, an endoscope using a CCD, and an endoscope using LED, as an optical system to be built-in. The light source is disposed on a control device side on the outside of the patient's body to guide light emitted from the light source by an optical fiber and irradiate the light from the tip portion of the endoscope. An endoscope which can be used in the invention has, for example, a route (for example, a sub-lumen or a channel) different from the optical system, and is capable of performing local cleaning, injection of gas and/or liquid, chemical spray, suction, treatment using a special treatment tool (device) and the like. Further, the direction of the tip of the endoscope can be freely changed by a hand operation using a means that is well known to those skilled in the art.

Further, in this specification, the term "proximal" refers to a portion of the tool and device on the side closer to the operator of the tool and device, and the term "distal" refers to a portion of the tool and device on the side away from the operator. Accordingly, the term "proximal end" represents an end portion that is closest (that is, proximal) to the operator, and the term "distal end" represents an end portion that is furthest (that is, distal) from the operator. The term "long axis direction" refers to a direction of axial center along the longitudinal direction of the over-tube.

As the endoscope which can be used in the invention, an endoscope of a suitable size is selected depending on the luminal organ as a target. As the luminal organ as the optional target, an esophagus, a stomach, a small intestine, a large intestine, a vagina, a bladder and the like can be cited.

As illustrated in Figs. 1(a) and 1(b), the over-tube device 100 for endoscope of the invention includes a tube body 110, a stopper ring 112, and a rotation lock ring 130.

In the invention, the tube body 110 is a tubular member in which a channel 134 for inserting the endoscope is formed therein. Although its shape is not particularly limited as long as the surface is smoothly formed so that the outer surface can rotate in the lumen of a patient, a substantially cylindrical shape is preferred, since the patient's burden is minimized when being rotated within the lumen.

The outer diameter of the tube body 110 is also not particularly limited as long as an excessive burden is not applied to the lumen to which the over-tube device 100 is inserted, and since the optimal range also differs depending on the type of the lumen to be inserted and the age of the patient, it cannot be said sweepingly, and for example, when directed to the digestive organs of adult male, an outer diameter may be preferably 20 mm or less, more preferably, 18 mm or less, and most preferably, 15 to 18 mm.

The material of the tube body 110 is not particularly limited, as long as the material has a suitable flexibility, lack of friction (lubricity), strength, column stiffness and the like, and is generally used for medical devices. As polymer used in such a medical device, soft resin such as polyvinyl chloride, silicone and polyurethane is adopted. From the viewpoint of small friction, polyvinyl chloride is preferred.

As illustrated in Figs. 1(a) and 1(b), a channel 134 is formed inside the tube body 110. Its diameter is not particularly limited as long as the insertion of the endoscope is allowed. In an embodiment, when the diameter of the endoscope is 11.8 mm diameter, the diameter of the channel 134 is preferably 12 mm or more, and for example, when the endoscope having a diameter of 5 mm is used as an endoscope of an ultrafine diameter, the channel 134 having the diameter slightly larger than 5 mm is adopted. In the invention, the endoscope is inserted into the channel 134 from the proximal end, and the distal end tip portion of the endoscope can protrude from the distal end.

Further, in order to allow a variety of surgical procedures, the tube body 110 preferably includes a second channel 114 that is configured to allow the insertion of the treatment tool. Further, in order to minimize the burden on the patient, as illustrated in Figs. 1(a) and 1(b), the second channel 114 is preferably provided to protrude to the channel 134 side rather than protruding to the outside of the tube body 110. However, the second channel 114 can also protrude to the outside in a range that does not damage the patient's lumen. In the example illustrated in Fig. 1(a) to Fig. 4, the second channel 114 extends substantially parallel to the channel 134, but is not limited thereto, for example, the second channel 114 may be provided in a spiral shape with the long axis direction of the channel 134 as a central axis.

The diameter of the second channel 114 is not particularly limited, as long as its size allows the insertion of the treatment tool, and may be suitably determined in accordance with the treatment tool to be used, but is preferably in a range from 2.8 mm to 3 mm. In the invention, the second channel 114 is used such that the treatment tool is typically inserted from the proximal end, and the treatment tool protrudes from the distal end. Further, since the second channel 114 is a lumen that is independent from the channel 134, it is possible to insert various treatment tools, independently of the endoscope that is inserted into the channel 134.

A handling connector 150 is preferably provided at the proximal end of the tube body 110, for example, as illustrated in Figs. 2(a) and 2(b). The handling connector 150 is made of a resin harder than a soft resin that forms the tube body 110, in order to facilitate the insertion operation of the tool, such as a treatment tool and an endoscope. The shape and structure of the handling connector 150 are not particularly limited, and it is possible to preferably use any shape and structure of the handling connector that can be generally used in the conventional over-tube device for endoscope.

Further, in the example illustrated in Figs. 2 (a) and 2(b), an opening portion 152 and a forceps port 118 are provided at the proximal end of the handling connector 150. The proximal end of the tube body 110 is adhered to the distal end that is in communication with the opening portion 152 of the handling connector 150, and is configured to cause the endoscope inserted through the opening portion 152 to protrude from the distal end of the tube body 110. The proximal end of the second channel 114 is adhered to the forceps port 118 of the handling connector 150 spaced apart from the opening portion 152 of the handling connector 150 by 25 mm between the centers, and is configured such that the treatment tool inserted from the forceps port 118 protrudes from the distal end of the second channel 114.

In the invention, as illustrated in Fig. 1(a) and Fig. 3(a), a stopper ring 112 is mounted to surround the side portion of the tube body 110. The shape of the stopper ring 112 is not particularly limited as long as the stopper ring 112 can be mounted to surround the tube body 110, but at least the outer periphery thereof has preferably a circular shape such that a rotation lock ring 130 to be described later can smoothly rotate with respect to the tube body 110.

In the example illustrated in Figs. 3(a) and 3(b), the stopper ring 112 is made up of two members, has a structure that is hooked to each other by male and female claws, and is adhered to the tube body. Further, the stopper ring 112 is a member for determining an end point of the distal end when, the rotation lock ring 130 to be described later is made to slide along the tube body 110. Thus, the stopper ring 112 comes into close contact with the tube body 110 so as not to move during use of the endoscope, and its position is preferably tightly fixed.

The position to which the stopper ring 112 is mounted cannot be said sweepingly because the position differs depending on the patient's physique and the operative procedure of the surgical procedure performed. However, in the case of the esophagus ESD surgery of the general adult male, the position can be set so that a slide movable range of the rotation lock ring is, for example, in a range from 50 mm to 100 mm.

As illustrated in Figs. 1(a) and 1(b), in the invention, the rotation lock ring 130 is mounted to surround the side portion of the tube body 110 and to cover the stopper ring 112. As illustrated in Figs. 2(a) and 2(b), the rotation lock ring 130 can be made to slide in the axial direction of the tube body 110.

Although the size of the rotation lock ring 130 is not particularly limited, it is preferred to set the size to the extent that the stopper ring 112 does not protrude from the bottom of the rotation lock ring 130 even when the rotation lock ring 130 is made to slide. In an embodiment, for example, when the slide movable range of the rotation lock ring 130 is 100 mm, the total length of the rotation lock ring 130 can be set to about 110 mm that is slightly longer than the slide movable range.

The inner diameter of the rotation lock ring 130 is not also particularly limited, as long as the inner diameter is enough to allow the rotation lock ring 130 to smoothly rotate around the tube body 110 and the stopper ring 112. In the embodiment illustrated in Fig. 1(a) to Fig. 4, for example, the diameter of the tube body 110 is set to 19 mm, the outer diameter of the stopper ring 112 is set to 22.3 mm, and the inner diameter of the rotation lock ring 130 is set to 23.6 mm. Accordingly, the rotation lock ring 130 can suitably rotate about the tube body 110.

The rotation lock ring 130 of the invention includes a connecting section 136 (see Figs. 1(a) and 1(b)) with the bite block, and a hook 138 (see Figs. 2(a) and 2(b), and Fig. 4).

The shape and structure of the connecting section 136 with the bite block provided on the rotation lock ring 130 is not particularly limited. As long as the effect of the invention is not impaired, the connecting section 136 that can be used for this type of over-tube device can also be entirely suitably used in the invention.

In the invention, the hook 138 extends from the base portion of the rotation lock ring 130, and the tip portion can be locked at the proximal end side of the stopper ring. With such a configuration, when the rotation lock ring 130 slides on the tube body 110 to the distal end side, if the rotation lock ring 130 reaches the distal end of the slide movable range, the tip side of the hook 138 abuts on the stopper ring 112 to be able to prevent the rotation lock ring 130 from further sliding to the distal end side.

The hook 138 may be a leaf spring in the invention. In this case, if the rotation lock ring 130 is inserted from the distal end side of the tube body 110, an inclined surface of the tip of the hook 138 comes into contact with the stopper ring 112 to warp upward, and gets over the stopper ring 112. Thus, since the hook 138 is locked at the proximal end side of the stopper ring 112, the mounting of the rotation lock ring 130 is facilitated. Further, since the leaf spring-shaped hook 138 returns to its original shape when getting over the stopper ring 112 once, it is not possible to pull out the tube body 110 to the original distal end side.

In the invention, a proximal end side stopper 120 may be provided at the proximal end of the tube body 110. If the proximal end side stopper 120 is provided, it is possible to prevent the rotation lock ring 130 from falling out of the proximal end side of the tube body 110. The shape of the proximal end side stopper 120 is not particularly limited, as long as falling-out of the rotation lock ring 130 can be prevented. Alternatively, the proximal end side stopper 120 may have a function other than the prevention of falling-out of the rotation lock ring 130. For example, in the examples illustrated in Fig. 1(a) to Fig. 3(b), the distal end side of the end cover that covers the aforementioned handling connector 150 functions as a proximal end side stopper 120. Further, in the examples illustrated in Fig. 1(a) to Fig. 3(b), the proximal end side stopper (end cover) 120 is inserted to the handling connector 150 with the stopper 116 provided at the proximal end of the handling connector 150 therebetween, and is fixed by being hooked with the claws.

Since the over-tube device for endoscope of the invention is configured as described above, it is possible to turn the tube body 110 or to extract and insert the tube body 110 front and back, in a state in which the rotation lock ring 130 is fixed to the patient's mouth via the bite block. Furthermore, the operator can prevent the extraction and insertion in an unintended range, by the stopper ring 112 and the proximal end side stopper 120.

That is, if the tube body 110 is pushed into the back of the patient's mouth, the rotation lock ring 130 relatively moves to the proximal end side of the tube body 110. However, if the tube body 110 is pushed to the limit, the distal end of the proximal end side stopper 120 and the proximal end of the rotation lock ring 130 abut on each other (in Fig. 2(a)). Thus, the tube body 110 cannot be further pushed to the patient's mouth.

Also, if the tube body 110 is pulled out of the patient's mouth, the rotation lock ring 130 relatively moves to the distal end side of the tube body 110. However, if the tube body 110 is pulled out to the limit, the proximal end of the stopper ring 112 and the hook 138 of the rotation lock ring 130 abut on each other (Fig. 2(b)). Thus, the tube body 110 cannot be further pulled out of the patient's mouth.

In the invention, by rotating the over-tube for endoscope itself, it is possible to rotationally move a treatment tool inserted into the second channel of the over-tube without rotating the endoscope (while keeping a constant visual field of the endoscope), the free extraction and insertion of the over-tube within the limited range are allowed, and a variety of surgical procedures such as endoscopic submucosal dissection (ESD) is allowed.

### <Second Example>

Next, a second example of the invention will be described. In the second example, an example in which the forceps port provided at the proximal end of the handling connector 150 is inclined with respect to an opening portion 152 will be described.

Figs. 5(a) and 5(b) illustrate an over-tube device 200 for endoscope in the present example. Fig. 5(a) is a side view of a case where the rotation lock ring 130 relatively moves to the proximal end side of the tube body 110, and Fig. 5(b) is a side view of a case where the rotation lock ring 130 relatively moves to the distal end side of the tube body 110.

The present example differs from the first example in that a forceps port 218 of a handling connector 250 in this example is obliquely provided. Figs. 6(a) and 6(b) illustrate cross-sectional views of the over-tube device 200 for endoscope. Fig. 6(a) is a cross-sectional view of a case where the rotation lock ring 130 relatively moves to the proximal end side of the tube body 110, and Fig. 6(b) is a cross-sectional view of a case where the rotation lock ring 130 relatively moves to the distal end side of the tube body 110.

As illustrated in Figs. 6(a) and 6(b), in this example, the forceps port 218 is inclined by approximately 20 degrees with respect to the opening portion 252 into which the endoscope is inserted, such that the axis of the forceps port 218 is spaced apart from the axis of the opening portion 252 toward the proximal side. According to this configuration, even when the endoscope is inserted into the over-tube device 200 for endoscope from the opening portion 252, and the treatment tool such as a forceps is inserted from the forceps port 218, the distance between the operating portions can be secured, and it is possible to prevent both the operating portions from interfering with each other. As a result, it is possible to perform the smoother insertion and extraction of the endoscope and the treatment tool, and it is possible to enhance the working efficiency of the endoscopic treatments.

The inclination angle of the forceps port 218 to the opening portion 252 in this example is approximately 20 degrees, but this angle is not limited to approximately 20 degrees. It is possible to suitably change the inclination angle, in accordance with the dimensions of the proximal end side stopper 220 and the handling connector 250, the dimension of the endoscope to be inserted into the opening portion 252, and the dimension of the treatment tool to be inserted from the forceps port 218. For example, the inclination angle may be in a range from 5 degrees to 75 degrees, and more preferably, may be in a range from 10 degrees to 40 degrees . By setting the inclination angles between them to these ranges, it is possible to more reliably enhance the working efficiency of endoscopic treatments.

In the present embodiment, a degassing prevention valve 218a is provided in the forceps port 218. The degassing prevention valve 218a has a structure in which a valve using an elastic body is provided in a portion corresponding to the opening of the forceps port 218, in a cap which is put on the forceps port 218. Further, in a state in which the treatment tool is not inserted into the forceps port 218, the overall air-tightness of the forceps port 218 is maintained, and in a state in which the treatment tool is inserted into the forceps port 218, the air-tightness of the gap between the forceps port 218 and the treatment tool is maintained. Thus, it is possible to prevent the gas supplied to the patient's body by the air supply from leaking via the second channel 114 and the forceps port 218.

Here, as in the case of the aforementioned forceps port 218, even in the opening portion 252 of the handling connector 250 of the invention, there is a concern that the supplied gas may flow backward via the endoscope and the channel 134 of the over-tube body 110, and may leak to the outside from the opening portion 252.

In contrast, in the invention, a degassing prevention adapter 160 can be mounted to the opening portion 252. The exterior of the degassing prevention adapter 160, for example, is the same as that illustrated in Figs. 7(a) to 7(d). Figs. 7(a) to 7(c) are side views of a degassing prevention adapter, and Fig. 7(d) is a plan view of the degassing prevention adapter. As illustrated in Fig. 7(a), the degassing prevention adapter 160 has a shape in which cylindrical shapes with gradually enlarged diameters, such as a tip portion 164, an adapter body 162, a cap portion 166, are combined.

Furthermore, as illustrated in Fig. 7(d), the degassing prevention adapter 160 has an insertion hole 162a as an opening into which an endoscope can be inserted, and a valve body 162b including a function of keeping the gap between the inner wall of the insertion hole 162a and the exterior of the endoscope in an airtight manner is provided inside the insertion hole 162a.

Furthermore, the degassing prevention adapter 160 has a stopcock body 168, and a switching lever 168a provided on the top of the stopcock body 168 to be rotatable, on the side surface of the adapter body 162. By rotating the switching lever 168a, it is possible to switch an opening state and a stopped state of the stopcock body 168, thereby being able to appropriately select the supply or degassing of gas to the patient during surgery.

When the degassing prevention adapter 160 is mounted to the opening portion 252, the tip portion 164 is inserted into the opening portion 252 such that the protruding portions 164a and 164b of two positions provided at the tip portion 164 are fitted to recessed portions 252a and 252b (to be described later) of the two positions provided in the opening portion 252. Thereafter, by rotating the degassing prevention adapter 160, the protruding portions 164a and 164b are moved along a recessed groove 252c (to be described later) provided on the inner wall of the opening portion 252. Thus, there is provided a structure in which the degassing prevention adapter 160 does not fall out of the opening portion 252 by merely axially pulling the degassing prevention adapter 160.

Next, the operation of the stopper 116 provided at the proximal end of the handling connector 250 will be described with reference to Figs. 8(a) and 8(b). Fig. 8(a) illustrates a state of the vicinity of the opening portion 252 of the handling connector 250 in a case where the stopper 116 is turned OFF. Fig. 8(b) illustrates a state of the vicinity of the opening portion 252 of the handling connector 250 in a case where the stopper 116 is turned ON. As can be seen from Fig. 8(a), in the state in which the stopper 116 is turned OFF, the lock member 116a is retracted from the recessed portion 252a to allow the connection between the recessed groove 252c and the recessed portion 252a.

In this state, when the degassing prevention adapter 160 mounted to the opening portion 252, for example, rotates with the rotation of the endoscope, in some cases, the protruding portion 164a is shifted to the recessed portion 252a from the recessed groove 252c, and may be further moved in the axial direction. As a result, there is a concern of detachment of the degassing prevention adapter 160 from the opening portion 252.

In contrast, as illustrated in Fig. 8(b), in the state in which the stopper 116 is made to slide to the proximal side and is turned ON, the lock member 116a protrudes to cut off between the recessed portion 252a and the recessed groove 252c. Thus, even when a rotational force or an axial force is applied to the degassing prevention adapter 160 in a state in which the protruding portion 164a and the recessed groove 252c are engaged with each other, it is possible to prevent a situation in which the protruding portion 164a is shifted to the recessed portion 252a from the recessed groove 252c and is further moved in the axial direction. As a result, it is possible to prevent detachment of the degassing prevention adapter 160 from the opening portion 252.

As described above, in this example, the forceps port 218 is inclined to the opening portion 252 so that the axis of the forceps port 218 and the axis of the opening portion 252 are spaced apart from each other toward the proximal side. Therefore, it is possible to perform smoother insertion and extraction of the endoscope and the treatment tool, and it is possible to enhance the working efficiency of the endoscopic treatments.

Further, in the present example, the degassing prevention valve 218a is provided in the forceps port 218. Thus, it is possible to prevent the gas supplied into the patient's body by the air supply from leaking via the second channel 114 and the forceps port 218.

Further, in the present example, the stopper 116 is provided at the proximal end of the handling connector 250. Thus, it is possible to prevent detachment of the degassing prevention adapter 160 from the opening portion 252 in the state in which the stopper 116 is turned ON.

In the present example, the forceps ports 118 and 218 correspond to the treatment tool insertion ports. Further, the opening portions 152 and 252 correspond to the endoscope insertion ports. Further, in this example, the lock mechanism includes the stopper 116 and the lock member 116a.

### Industrial Applicability

According to the invention, it is possible to allow the free extraction and insertion of the over-tube within the limited range, while maintaining the rotation ability of the over-tube, and it is possible to prevent extraction and insertion of the over-tube of an unintended range of the operator. The over-tube device for endoscope of the invention, for example, is useful for overall endoscopic therapies, such as an endoscopic submucosal dissection (ESD), in which simplification and reduction of the procedure can be expected, by applying adequate traction to the tissue.

### Reference Signs List

100, 200 over-tube device for endoscope
110 tube body
112 stopper ring
114 second channel
116 stopper
118, 218 forceps port
120, 220 proximal end side stopper (end cover)
130 rotation lock ring
132 leaf spring
134 channel
136 connecting section
138 hook
150, 250 handling connector
152, 252 opening portion
218a degassing prevention valve

## Claims

1. An over-tube device for an endoscope (100, 200) comprising:
a tube body(110) that has a smooth outer surface capable of rotating within a lumen of a patient, and includes a proximal end side stopper(120,220) at a proximal end and a channel(134) for inserting the endoscope thereinto;
a stopper ring(112) mounted to surround a side portion of the tube body; and
a rotation lock ring(130) that is mounted to surround the side portion of the tube body and to cover the stopper ring, and is capable of sliding in an axial direction of the tube body,
the rotation lock ring including:
a connecting section(136) for being connected to a bite block; and
a hook(138) extending from a base portion, that is capable of being locked on a proximal end side of the stopper ring,
the over-tube device **characterised in that** the hook of the rotation lock ring is slidable from the stopper ring to the proximal end side stopper.

2. The device according to claim 1, wherein
an outer surface of the tube body has a substantially cylindrical shape.

3. The device according to claim 1 or 2, wherein
the hook extending from the base portion of the rotation lock ring constitutes a leaf spring(132).

4. The device according to any one of claims 1 to 3, wherein
the tube body includes a second channel(114) for inserting a treatment tool.

5. The device according to 4, wherein
the proximal end side stopper has a treatment tool insertion port(118,218) as an opening capable of inserting the treatment tool into the second channel by inserting the treatment tool, and an endoscope insertion port(152,252) as an opening capable of inserting the endoscope into the channel by inserting the endoscope, and
the treatment tool insertion port and the endoscope insertion port are provided to be inclined to each other so that a gap between axes thereof becomes large toward the proximal side.

6. The device according to claim 5, wherein
the treatment tool insertion port is provided with a degassing prevention valve(218a) that prevents degassing through the second channel.

7. The device according to any one of claims 1 to 3, wherein
the proximal end side stopper has an endoscope insertion port(152,252) as an opening capable of inserting the endoscope into the channel by inserting the endoscope, and the endoscope insertion port is configured to mount a degassing prevention adapter(160) for preventing degassing through the channel, and has a lock mechanism that prevents the mounted degassing prevention adapter from being detached from the endoscope insertion port.

## Patentansprüche

1. Überrohr-Vorrichtung für ein Endoskop (100, 200), umfassend:
einen Rohrkörper (110), der eine glatte Außenfläche hat, die innerhalb eines Lumens eines Patienten rotieren kann, und umfasst einen proximalen endseitigen Stopper (120, 220) an einem proximalen Ende und einen Kanal (134) zum Einführen des Endoskops darin;
einen Stopperring (112), der so montiert ist, dass er einen Seitenteil des Rohrkörpers umgibt; und
einen Rotations-Sperrring (130), der so montiert ist, dass er den Seitenteil des Rohkörpers umgibt und den Stopperring abdeckt, und der in der Lage ist, in einer axialen Richtung des Rohkörpers zu gleiten,
wobei der Rotations-Sperrring umfasst:
einen Verbindungsabschnitt (136) zum Verbinden mit einem Beißblock; und
einen Haken (138), der sich von einem Basisteil aus erstreckt, der in der Lage ist, an einer proximalen Endseite des Stopperrings gesperrt zu werden,
wobei die Überrohr-Vorrichtung **dadurch gekennzeichnet ist, dass** der Haken des Rotations-Sperrrings vom Stopperring zum proximalen Endseiten-Stopper verschiebbar ist.

2. Vorrichtung nach Anspruch 1, wobei
eine Außenfläche des Rohkörpers eine im Wesentlichen zylindrische Form hat.

3. Vorrichtung nach Anspruch 1 oder 2, wobei
der Haken, der sich vom Basisteil des Rotations-Sperrrings aus erstreckt, eine Blattfeder (132) darstellt.

4. Vorrichtung nach einem der Ansprüche 1 bis 3, wobei
der Rohrkörper einen zweiten Kanal (114) zum Einführen eines Behandlungswerkzeugs umfasst.

5. Vorrichtung nach Anspruch 4, wobei
der proximale endseitige Stopper einen Behandlungswerkzeug-Einführungsport (118, 218) als Öffnung hat, die in der Lage ist, das Behandlungswerkzeug in den zweiten Kanal einzuführen, durch Einführen des Behandlungswerkzeugs, und einen Endoskop-Einführungsport (152, 252) als Öffnung, die in der Lage ist, das Endoskop in den Kanal einzuführen durch Einführung des Endoskops, und
der Behandlungswerkzeug-Einführungsport und der Endoskop-Einführungsport sind so vorgesehen, dass sie zueinander geneigt sind, sodass ein Spalt zwischen den Achsen derselben zur proximalen Seite hin groß wird.

6. Vorrichtung nach Anspruch 5, wobei
der Behandlung Werkzeug-Einführungsport mit einem Entgasungs-Verhinderungsventil (218a) versehen ist, welches das Entgasen durch den zweiten Kanal verhindert.

7. Vorrichtung nach einem der Ansprüche 1 bis 3, wobei
der proximale endseitige Stopper einen Endoskop-Einführungsport (152, 252) als Öffnung hat, die in der Lage ist, dass Endoskop in den Kanal einzuführen durch Einführen des Endoskops, und der Endoskop-Einführungsport ist dafür ausgelegt, einen Entgasungs-Verhinderungs-Adapter (160) zum Verhindern des Entgasens durch den Kanal zu montieren, und hat einen Sperrmechanismus, der verhindert, dass der montierte Entgasungs-Verhinderungs-Adapter vom Endoskop-Einführungsport getrennt wird.

## Revendications

1. Dispositif sur-tube pour un endoscope (100, 200) comprenant :
un corps de tube (110) qui a une surface externe lisse capable de tourner à l'intérieur d'une lumière d'un patient, et comprend une butée (120, 220) du côté de l'extrémité proximale au niveau d'une extrémité proximale et un canal (134) pour y insérer l'endoscope ;
une bague de butée (112) montée pour entourer une partie latérale du corps de tube ; et
une bague de blocage de rotation (130) qui est montée pour entourer la partie latérale du corps de tube et pour recouvrir la bague de butée, et peut coulisser dans une direction axiale du corps de tube,
la bague de blocage de rotation comprenant :
une section de raccordement (136) pour être raccordée à un bloc de morsure ; et
un crochet (138) s'étendant à partir d'une partie de base, qui peut être bloqué sur un côté d'extrémité proximale de la bague de butée,
le dispositif sur-tube étant **caractérisé en ce que** le crochet de la bague de blocage de rotation peut coulisser de la bague de butée à la butée du côté de l'extrémité proximale.

2. Dispositif selon la revendication 1, dans lequel :
une surface externe du corps de tube a une forme sensiblement cylindrique.

3. Dispositif selon la revendication 1 ou 2, dans lequel :
le crochet s'étendant à partir de la partie de base de la bague de blocage de rotation constitue un ressort à lames (132).

4. Dispositif selon l'une quelconque des revendications 1 à 3, dans lequel :
le corps de tube comprend un second canal (114) pour insérer un outil de traitement.

5. Dispositif selon la revendication 4, dans lequel :
la butée du côté de l'extrémité proximale a un orifice d'insertion d'outil de traitement (118, 218) en tant qu'ouverture pouvant insérer l'outil de traitement dans le second canal en insérant l'outil de traitement, et un orifice d'insertion d'endoscope (152, 252) en tant qu'ouverture pouvant insérer l'endoscope dans le canal en insérant l'endoscope, et
l'orifice d'insertion d'outil de traitement et l'orifice d'insertion d'endoscope sont prévus pour être inclinés l'un par rapport à l'autre de sorte qu'un espace entre leurs axes s'élargit vers le côté proximal.

6. Dispositif selon la revendication 5, dans lequel :
l'orifice d'insertion d'outil de traitement est prévu avec une valve de prévention de dégazage (218a) qui empêche le dégazage par le second canal.

7. Dispositif selon l'une quelconque des revendications 1 à 3, dans lequel :
la butée du côté de l'extrémité proximale a un orifice d'insertion d'endoscope (152, 252) en tant qu'ouverture capable d'insérer l'endoscope dans le canal en insérant l'endoscope, et l'orifice d'insertion d'endoscope est configuré pour monter un adaptateur de prévention de dégazage (160) pour empêcher le dégazage à travers le canal, et a un mécanisme de blocage qui empêche l'adaptateur de prévention de dégazage monté de se détacher de l'orifice d'insertion d'endoscope.
